# EUROPÄISCHE PATENTANMELDUNG

(11) **EP 0 652 225 A1**
(43) Veröffentlichungstag der Anmeldung: **10.05.1995**
(21) Anmeldenummer: 94116355.2
(22) Anmeldetag: 17.10.1994
(51) Int. Cl.: C07H 15/04, C07D 309/08, C07K 7/02, C07K 7/56, C07K 7/23, A61K 38/09

(54) **Verwendung von D-glucopyranuronsäuren und deren Derivate zum Einbau in pharmakologisch wirksame Peptide und deren Salze**

(30) Priorität: 08.11.1993 DE 4338015
(71) Anmelder: ASTA Medica Aktiengesellschaft, D-01277 Dresden (DE)
(72) Erfinder: Graf v. Roedern, Erich, D-63628 Bad Soden-Salzmünster (DE); Kessler, Horst, Prof., D-65824 Schwalbach (DE); Kutscher, Bernhard, Dr., D-63477 Maintal (DE); Bernd, Michael, Dr., D-60316 Frankfurt (DE); Klenner, Thomas, Dr., D-69493 Kirschberg (DE)

(57) **Zusammenfassung**

Verwendung von 7-Amino-L-glycero-L-gulo-2,6-anhydro-7-desoxy-heptonsäure und 2-Benzoxycarbonylamino-0¹-methyl-2-desoxy-β-glucopyranuronsäure, ihrer Derivate und der enantiomeren Verbindungen zum Einbau und zur Herstellung in pharmakologisch wirksame Peptidhormone.

## Beschreibung

Die Erfindung betrifft 7-Amino-L-glycero-L-gulo-2,6-anhydro-7-desoxy-heptonsäure, ein mehrstufiges Verfahren zur Herstellung sowie die Verwendung der Verbindung und der enantiomeren 7-Amino-D-glycero-D-gulo-2,6-anhydro-7-desoxy-heptonsäure zum Einbau in pharmakologisch wirksame Peptidhormone.

Weiterhin betrifft die Erfindung 2-Benzoxycarbonylamino-O¹-methyl-2-desoxy-β-D-glucopyranuronsäure sowie die Verwendung der Verbindung und der enantiomeren 2-Benzoxycarbonylamino-O¹-methyl-2-desoxy-α-D-glucopyranuronsäure zum Einbau in pharmakologisch wirksame Petidhormone.

In den letzten Jahrzehnten wurden zahlreiche biologisch aktive Peptide entdeckt und charakterisiert. Als Neurotransmitter, Neuromodulatoren und Hormone beeinflussen sie nach Bindung an ihre membranständigen Rezeptoren die Zell-Zell Kommunikation und kontrollieren eine Reihe vitaler Funktionen wie Stoffwechsel, Immunabwehr, Verdauung, Atmung, Schmerzempfindung usw.
Aufgrund der vielfältigen Funktionen sind sie von großem medizinischen Interesse, so daß die Zahl der nativen und modifizierten Peptide, die als Medikamente eingesetzt werden, ständig ansteigt. Von besonderem Interesse ist die Möglichkeit, durch den Einbau beziehungsweise durch den Austausch von einzelnen Aminosäuren in der Peptidkette die konformativen und biologischen Parameter zu verändern.

Die auf diese Weise modifizierten Peptide können veränderte biochemische und pharmakologische Eigenschaften aufweisen (A. Giannis, T. Kolter Angew. Chem. 1993 105 1303). Beim Design von modifizierten Peptiden bieten Kohlenhydrate den Vorteil der Strukturvielfalt und der physiologischen Unbedenklichkeit. Durch den Einbau von spezifischen Zuckermolekülen in Peptidhormone kann deren pharmakologische Aktivität wesentlich verändert werden.

Gegenstand der Erfindung ist die Bereitstellung der neuen Zuckeraminosäure 7-Amino-L-glycero-L-gulo-2,6-anhydro-7-desoxy-heptonsäure. 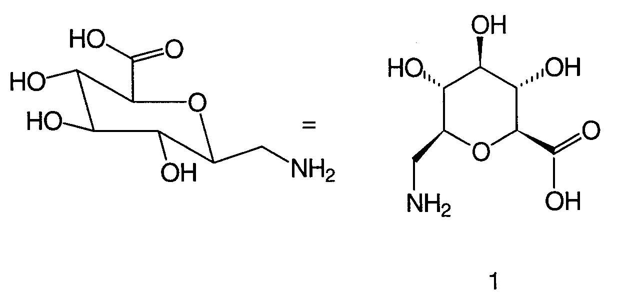
Die Verbindung besitzt als Strukturmerkmal das starre, pyranoide Grundgerüst des Zuckers. Dieses schränkt die Konformation von Peptiden ein. Die freien Hydroxylgruppen erhöhen die Hydrophilie der Peptide, andererseits kann durch Verwendung unpolarer Schutzgruppen leicht der gegenteilige Effekt erzielt werden.
Während pyranoide Ringe in therapeutisch relevanten Naturstoffen häufig vorkommen, ist ein solches Strukturmerkmal innerhalb einer Peptidkette nur für den Fall der Galatinsäure, welche Bestandteil des natürlichen Peptids Galatin I ist, bekannt (N. Sakai, Y. Ohfume J. Am. Chem. Soc. 1992 114 998-1010).

Die Zuckeraminosäure 7-Amino-L-glycero-L-gulo-2,6-anhydro-7-desoxy-heptonsäure ist über vier Stufen von D-Glucose aus in einer Gesamtausbeute von 12 % erhältlich.

Durch Kondensation von Nitromethan an Glucose wird in an sich bekannter Weise die Nitroverbindung 2 erhalten (L. Petrus, S. Bystricky, T. Sticzay, V. Bilik Chem. zvesti 1982, 36(1) 103-110). Diese wird mit Wasserstoff an einem Palladium-Katalysator quantitativ zum Amin reduziert, und anschließend wird die Aminogruppe durch Umsetzung mit Chlorameisensäurebenzylester durch die Einführung der Benzyloxy-Carbonylgruppe geschützt. Durch selektive Oxidation der Verbindung 3 mit Sauerstoff an einem Platin-Katalysator wird die primäre Hydroxygruppe zur Carbonsäure oxidiert und anschließend mit Methanol zum Methylester 4 umgesetzt. Durch Entfernen der Schutzgruppen in an sich bekannter Weise kann die freie Aminosäure 1 kristallin erhalten werden. 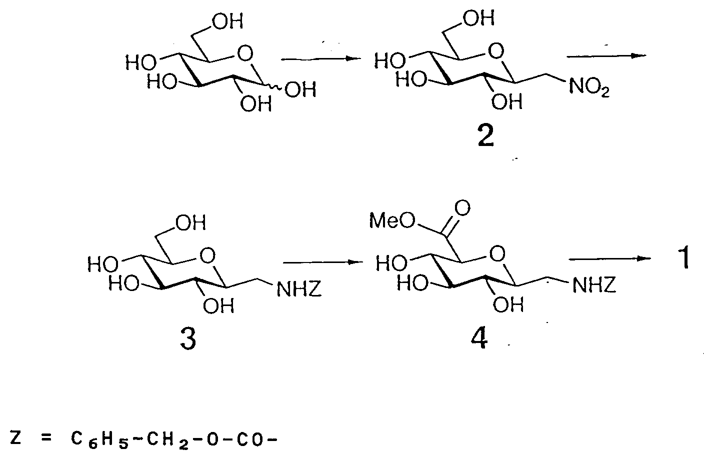
Die preisgünstigen Einsatzstoffe sowie die wiederverwertbaren Palladium- und Platin-Katalysatoren erlauben eine Synthese in technischem Maßstab.

Die enantiomere Zuckeraminosäure 7-Amino-D-glycero-D-gulo-2,6-anhydro-7-desoxy-heptonsäure ist Literaturbekannt (E.F. Fuch, J. Lehmann (Chem. Ber. 108 (1975) 2254).

Die Synthese der Verbindung erfolgt ebenfalls ausgehend von D-Glucose, jedoch sehr aufwendig über zehn Stufen.
Über die mögliche Verwendung dieser Verbindung als Peptidbaustein wird keine Aussage getroffen.

Weiterhin können die beiden Zuckeraminosäuren 2-Benzoxycarbonylamino-O¹-methyl-2-desoxy-α-D-glucopyranuronsäure 5 und 2-Benzoxycarbonylamino-O¹-methyl-2-desoxy-β-D-glucopyranuronsäure 6 und/oder deren ungeschützte Derivate in pharmakologisch wirksame Peptide eingebaut werden. 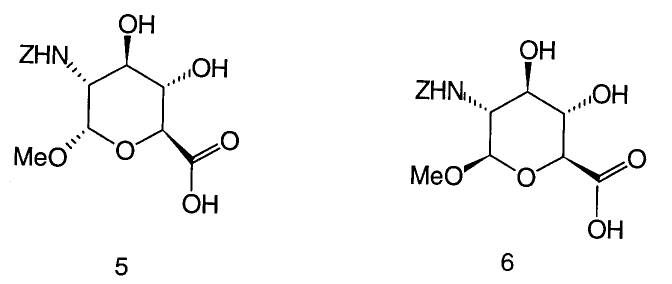
Die Verbindung 5 ist literaturbekannt (K.Heyns, H.Paulsen Chem. Ber. 1953, 88, 188 - 195), jedoch wird über eine mögliche Verwendung der Verbindung keine Aussage getroffen.

Die neue Verbindung 6 laßt sich ausgehend von 2-Amino-O¹-Methyl-2-desoxy-Tri-O³,O⁴,O⁶-acetyl-β-D-gluco pyranosid Hydrobromid 8 herstellen (G. Fodor, L. Ötvös, Chem. Ber. 1955, 89, 701 - 708). Durch Umsetzung mit Chloramelsensäurebenzylester und anschließender Reaktion mit Dimethylethylamin in Methanol erhält man 2-Benzyloxy-carbonylamino-0¹-methyl-2-desoxy-β-D-glucopyranosid 7.

Die Oxidation der Verbindung 7 mit Sauerstoff an einem Platinkatalysator ergibt schließlich 2-Benzoxycarbonylamino-O¹-methyl-2-desoxy-β-D-glucopyranuronsäure 6 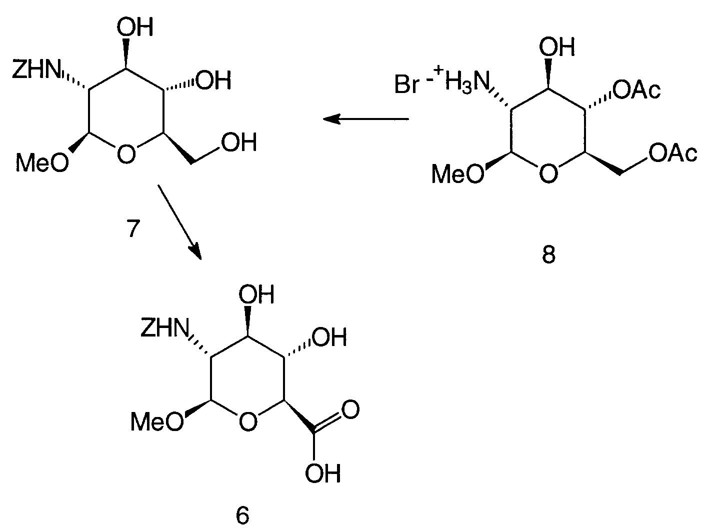
Die enantiomeren Zuckeraminosäuren können als Aminosäureersatz innerhalb der Peptidkette eingesetzt werden, so daß neue derivatisierte Peptidhormone mit agonistischen, aber auch antagonistischen Eigenschaften hergestellt werden können.

7-Amino-L-glycero-L-gulo-2,6-anhydro-7-desoxy-heptonsäure kann beispielsweise in cyclische Hexapeptide in der Weise eingebaut werden, daß jeweils eine oder zwei benachbarte Aminosäuren ersetzt werden. Es wurden folgende cyclische Peptide hergestellt, die eine Aktivität als Somatostatin-Analoga aufweisen:
cyclo(-Zu¹-Tyr²-D-Trp³-Lys⁴-Val⁵) D-22475
cyclo(-Zu¹-Phe²D-Trp³-Lys⁴-Thr⁵) D-22402
- Zu =: 7-Amino-L-glycero-L-gulo-2,6-anhydro-7-desoxy-heptonsäure
Die Peptide leiten sich von cyclischen Somatostatin-Analoga ab (D.F. Veber Peptides, Chemistry and Biology, Proc. of the Twelfth American Peptide Symposium (Hrsg.: J.A. Smith, J.E. Rivier) Escom, Leiden, 1992, S. 3-14), die anstelle über Zu über die Aminosäuren -Pro-Phe- oder eine Cystin-Disulfidbrücke cyclisiert sind.

7-Amino-L-glycero-L-gulo-2,6-anhydro-7-desoxy-heptonsäure kann ebenfalls in langkettige nichtcyclische Peptide eingebaut werden. Beispielsweise kann folgender LH-RH-(Luteintzing Hormone Releasing Hormone) Antagonist erhalten werden.
Ac-D-(2-NaL)-p-Cl-D-Phe-D-(3-Pal)-Ser-Tyr-D-Lys-(Zu)-Leu-Arg-Pro-D-AlaNH₂ x TFA D-22620
- Zu =: 7-Amino-L-glycero-L-gulo-2,6-anhydro-7-desoxy-heptonsäure
- TFA =: Trifluoracetat
Dieser Antagonist zeigt eine langhaltende Testosteron-Suppression in der Dosierung 0,5 - 1,5 mg/kg im Tierversuch bei Ratten.

Durch Einbau der geschützten Zuckeraminosäure 7-Benzoxycarbonylamino-L-glycero-L-gulo-2,6-anhydro-7-desoxyheptonsäuremethylester in den vom Nafarelin abgeleiteten LHRH-Agonisten läßt sich beispielsweise pGlu-His-Trp-Ser-Zu-Leu-Arg-Pro-Gly-NH₂ x 2 TFA D-22677 erhalten.

Durch Einbau der obengenannten geschützten Zuckeraminosäure in den vom Cetrorelix abgeleiteten LHRH-Antagonisten läßt sich Ac-D-(2-Nal)-p-Cl-D-Phe-D-(3-Pal)-Ser-Tyr-Zu-Leu-Arg(TFA)-Pro-D-Ala-NH₂ D-23010 und Ac-D-(2-Nal)-p-Cl-D-Phe-D-(3-Pal)-Ser-Zu-Leu-Arg(TFA)-Pro-D-Ala-NH₂ ER 133 erhalten.

Durch Verwendung von hydrophileren oder stärker hydrophoberen Schutzgruppen in der neuen Zuckeraminosäure kann beim Einbau in Peptide und in deren Salze die pharmakologische Wirksamkeit gesteuert werden. So kann beispielsweise durch Acetylierung 7-Acetylamino-L-glycero-L-gulo-2,6-anhydro-7-desoxy-heptonsäuremethylester hergestellt werden, der ebenfalls in Peptide eingebaut werden kann.

### Darstellung von β-D-Glucopyranosylnitromethan (2)

50 g wasserfreie D-Glucose werden in 200 ml Dimethylsulfoxid und 100 ml Methanol gelöst. Hierzu gibt man 100 ml Nitromethan und eine Suspension von 30 g Natriummethylat in 320 ml Methanol und rührt 24 Stunden bei Raumtemperatur. Zur Reaktionsmischung werden 250 ml n-Butanol zugegeben und für 1 Stunde bei 0° C gerührt.
Man filtriert und wäscht den Rückstand mit 100 ml Isopropanol und 100 ml Diethylether. Nach dem Trocknen erhält man 71 g eines hellgelben Pulvers. Dieses wird in 200 ml Wasser gelöst und über eine Säule mit 100 g Kationenaustauscher (Aldrich, Amberlyst 15, stark sauer H⁺-Form) gegeben. Der Ionenaustauscher wird mit 500 ml Wasser gewaschen. Die wäßrige, rote Lösung wird auf 100 ml eingeengt und 30 Stunden auf 90° C erhitzt. In die heiße Lösung gibt man 2 g feine Aktivkohle, rührt 10 Minuten, filtriert über 5 g Kieselgur und wäscht mit 200 ml Wasser nach.

Zu der Lösung gibt man 150 g Anionenaustauscher (Fluka, Amberlyst A-26, stark basisch OH⁻-Form) und rührt 1 Stunde. Man filtriert und wäscht den Ionenaustauscher mit 3 - 5 l Wasser. Der Ionenaustauscher wird hierauf in 400 ml Wasser gegeben, in welches man für 30 Minuten Kohlendioxidgas einleitet, um das Produkt freizusetzen.
Der Ionenaustauscher wird abfiltriert und mit 1000 ml Wasser gewaschen.

Das Filtrat wird im Vakuum auf 30 ml eingeengt und lyophilisiert. Der so erhaltene harte Schaum wird mehrfach aus Aceton umkristallisiert. Man erhält 19,3 g Produkt als farbloses Pulver
(Schmp.: 175° C [α_{D}] = + 9.1°) Lit.: L. Petrus, S. Bystricky, T. Sticzay, V. Bilik Chem. zvesti 1982, 36(1), 103-110.

### Darstellung von N-Benzyloxycarbonyl-β-D-glucopyranosylmethylamin (3)

5 g β-D-Glucopyranosylnitromethan (2) werden in 80 ml Methanol und 20 ml Wasser gelöst, 3 g Palladium-Katalysator (Degussa, Pd-C 10%ig, ca. 50 % Wasser) zugegeben und 24 Stunden unter Wasserstoff-Atmosphäre gerührt (Wasserstoff-Verbrauch ca. 3,5 l). Der Katalysator wird abfiltriert und mit einem Gemisch aus 80 ml Wasser und 20 ml Methanol gewaschen. Zu dieser Lösung werden 10,5 g Natriumhydrogencarbonat und 17 ml Chlorameisensäurebenzylester gegeben und 1 Stunde kräftig gerührt. Man gibt zur Reaktionsmischung 50 ml Wasser und entfernt das Methanol im Vakuum. Die wäßrige Lösung wird drei mal mit je 20 ml Diethylether gewaschen und im Vakuum zur Trockne eingedampft (Badtemperatur 40° C). Der Rückstand wird in 80 ml Aceton aufgenommen.
Die anorganischen Salze werden abfiltriert und portionsweise mit 80 ml Aceton gewaschen. Das Aceton wird im Vakuum destillativ entfernt, nach dem Trocknen erhält man 7,21 g dünnschicht-chromatographisch einheitliches Produkt als farblosen harten Schaum.

### Darstellung von 7-Benzyloxycarbonylamino-L-glycero-L-gulo 2,6-anhydro-7-desoxy-heptonsäuremethylester (4)

5 g (N-Benzoxycarbonyl)-β-D-Glucopyranosylmethylamin(3) werden in 100 ml Wasser gelöst und 5 g Platin-Katalysator (Degussa, Pt-C 10 %ig, ca. 50 % Wasser, Aktivierung durch siedenden Eisessig) zugegeben.

Bei 90° C wird 36 Stunden unter Rühren ein starker Sauerstoffstrom eingeleitet. Der pH-Wert wird durch stündliche Zugabe von 10 %iger Natriumhydrogencarbonat-Lösung zwischen 7 und 8 gehalten. Nach 8 und 18 Stunden werden abermals 2 g Platin-Katalysator zugegeben. Der Katalysator wird abfiltriert und mit 30 ml Wasser gewaschen. Zu der wäßrigen Lösung werden 10 g Kationenaustauscher (Aldrich, Amberlyst 15, stark sauer, H⁺-Form) gegeben und 10 Minuten gerührt. Der Ionenaustauscher wird abfiltriert und mit Wasser gewaschen, das Filtrat im Vakuum eingeengt und lyophilisiert. Der pulvrige Rückstand wird in 100 ml absolutem Methanol gelöst, 2,9 g Dicyclohexylcarbodiimid sowie 0,2 g 4-(Dimethylamino)-pyridin zugegeben und man rührt weitere 2 Stunden im Vakuum. Es wird fast bis zur Trockne eingedampft und der resultierende Sirup chromatographisch an Kieselgel (100 g Kieselgel, Laufmittel Chloroform : Methanol / 9 : 1) gereinigt. Von der Produktfraktion wird das Laufmittel destillativ entfernt, der erhaltene harte Schaum aus Wasser umkristallisiert. Man erhält 1,9 g dünnschichtchromatographisch einheitliches Produkt in Form farbloser Nadeln (Schmp.: 164° C, [α]_{D} = -21,7°).

### Darstellung von 7-Amino-L-glycero-L-gulo-2,6-anhydro-7-desoxy-heptonsäure (1)

0,1 g der geschützten Aminosäure 4 werden in 2 ml Methanol gelöst, 0,4 ml 1N Natronlauge zugegeben und 5 Minuten gerührt.

Nach Zugabe von 0,3 g Kationenaustauscher (Aldrich, Amberlyst 15, stark sauer, H⁺-Form) wird nochmals 5 Minuten gerührt, der Ionenaustauscher abfiltriert, und mit 2 ml Methanol gewaschen. Dem Filtrat werden 0,1 g Palladium-Katalysator (Degussa, Pt-C 10 %ig, ca. 50 % Wasser) zugesetzt und 4 Stunden unter Wasserstoff-Atmosphäre gerührt.

Der Katalysator wird abfiltriert, mit 2 ml Wasser und 2 ml Methanol gewaschen und das Lösungsmittel im Vakuum entfernt. Nach dem Trocknen erhält man 0,5 g Produkt als farbloses Pulver
(Schmp. > 260° C, [α]_{D} = -41,2°).

### N-Acetyl-β-D-glucopyranosylmethylamin

3,5 g β-D-Glucopyranosylnitromethan (2) werden in 80 ml MeOH und 20 ml H₂O gelöst, 3 g Pd-Katalysator (Degussa, Pd-C 10 %ig, ca. 50 % H₂O) zugegeben und 24 Stunden unter 50 bar H₂-Atmosphäre im Autoklaven gerührt (H₂-Verbrauch ca. 3,5 l). Der Katalysator wird abfiltriert und mit einem Gemisch aus 80 ml H₂O und 20 ml MeOH gewaschen und das Lösungsmittel im Vakuum entfernt. Der Rückstand wird in 50 ml abs. MeOH aufgenommen und mit 1,6 ml Acetanhydrid versetzt und 5 min. gerührt und das Lösungsmittel im Vakuum entfernt. Der Rückstand wird an Kieselgel (Laufmittel CHCl₃ : MeOH 4 : 1) chromatographiert. Nach dem Trocknen erhält man 3,5 g (95 %) Produkt als farblosen Schaum.

### 7-Acetylamino-L-glycero-L-gulo-2,6-anhydro-7-desoxy-heptonsäuremethylester

3 g N-Acetyl-β-D-Glucopyranosylmethylamin werden in 100 ml H₂O gelöst und 1,5 g Pt-Katalysator (Degussa, Pt-C 10 %ig, ca. 50 % H₂O, Aktivierung durch kurze Behandlung mit Ultraschall) zugegeben. Bei 90° C wird 22 Stunden unter Rühren ein starker O₂-Strom eingeleitet. Dieser wird mittels einer Pumpe in der geschlossenen Apparatur im Kreis gepumpt. Der pH-Wert wird durch stündliche Zugabe von 10 %iger NaHCO₃-Lösung zwischen 7 und 8 gehalten. Nach 8 Stunden und 18 Stunden werden abermals je 0,5 frisch aktivierter Pt-Katalysator zugegeben.

Nach Beendigung der Oxidation wird der Katalysator abfiltriert, zum Filtrat 10 g Kationenaustauscher (Aldrich, Amberlyst 15, stark sauer, H⁺Form) gegeben und 10 min. gerührt. Der Ionenaustauscher wird abfiltriert und gewaschen, das Filtrat im Vakuum eingeengt und lyophilisiert. Der pulvrige Rückstand wird in 100 ml abs. MeOH gelöst und 2,5 g DCC sowie 0,2 g 4-(Dimethylamino)-pyridin zugegeben und 0,5 Stunden gerührt. Das ausfallende Harnstoffderivat wird abfiltriert und mit MeOH gewaschen, das Filtrat wird im Vakuum fast bis zur Trockne eingeengt und der resultierende Sirup chromatographisch an Kieselgel (100 g Kieselgel, Laufmittel CHCl₃ : MeOH / 6 : 1) gereinigt. Man erhält 1,1 g (34 %) dünnschichtchromatographisch einheitliches Produkt in Form eines farblosen Schaums.

Die Herstellung von 2-Amino-O¹-Methyl-2-desoxy-tri-O³, O⁴,O⁶-acetyl-β-D-glucopyranosid Hydrobromid 8 erfolgt nach der Vorschrift von G. Fodor, L. Ötvös Chem. Ber. 1955, 89, 701 - 708.

### 2-Benzyloxycarbonylamino-O¹-Methyl-2-desoxy-β-D-glucopyranosid 7

56 g (0.66 mol) NaHCO₃ werden in einem 2 l Scheidetrichter in 500 ml H₂O gelöst, mit 700 ml Essigsäureethylester überschichtet und 70 ml (0.21 mol) einer 50 %igen Lösung von Chlorametsensäurebenzylester in Toluol hinzugegeben. 56 g (0.14 mol) 2-Amino-O¹-Methyl-2-desoxy-tri-O³,O⁴,O⁶-acetyl-α-D-glucopyranosid Hydrobromid 8 werden in 100 ml H₂O gelöst und vorsichtig zu dieser Mischung gegeben. Es wird vorsichtig geschüttelt und nachdem keine CO₂-Entwicklung mehr feststellbar ist, weitere 5 min. geschüttelt. Die wäßrige Phase wird abgetrennt, die org. Phase 1 mal mit 100 ml H₂O, drei mal mit je 100 ml 1.0 HCl und drei mal mit je 100 ml H₂O gewaschen.

Die org. Phase wird mit MgSO₄ getrocknet und das Lösungsmittel im Vakuum restlos entfernt. Man erhält 62 g farblose Plättchen, welche in 150 ml abs. MeOH gelöst werden, hierzu gibt man 20 ml Dimethylethylamin und läßt über Nacht stehen. Man entfernt das Lösungsmittel im Vakuum und kristallisiert aus Wasser um. Man erhält 44 g (96 %) 2-Benzoxycarbonylamino-O¹-methyl-2-desoxy-β-D-glucopyranosid 7 als feine farblose Nadeln.

### 2-Benzyloxycarbonylamino-O¹-methyl-2-desoxy-β-D-glucopyranuronsäure 6

10 g 2-Benzoxycarbonylamino-O¹-methyl-2-desoxy-β-D-glucopyranosid 7 werden in 100 ml H₂O gelöst und 5 g Pt-Katalysator (Degussa, Pt-C 10 %ig, ca. 50 % H₂O, Aktivierung durch kurze Behandlung mit Ultraschall) zugegeben. Bei 85° C wird 50 Stunden unter Rühren ein starker O₂-Strom eingeleitet. Dieser wird mittels einer Pumpe in der geschlossenen Apparatur im Kreis gepumpt.
Der pH-Wert wird durch stündliche Zugabe von 10 %iger NaHCO₃-Lösung zwischen 7 und 8 gehalten. Nach 15 Stunden und 30 Stunden werden abermals je 2 frisch aktivierter Pt-Katalysator zugegeben. Nach Beendigung der Oxidation wird der Katalysator abfiltriert, zum Filtrat 20 g Kationenaustauscher (Aldrich, Amberlyst 15, stark sauer, H⁺-Form) gegeben und 10 min. gerührt. Der Ionenaustauscher wird abfiltriert und mit Wasser gewaschen, das Filtrat im Vakuum auf ca. 30 ml eingeengt, wobei 5,2 g (54 %) 2-Benzoxycarbonylamino-O¹-methyl-2-desoxy-β-D-glucopyranuronsäure 6 in Form farbloser feiner Nadeln auskristallisiert (Schmp.: 142° C).

### Darstellung von cyclo-(-Zu¹-Phe²-D-Trp³-Lys⁴-Thr⁵) D-22402

Die Synthese wurde in flüssiger Phase durchgeführt.
Bei den Peptid-Kupplungen wurde mit EDCI (1-Ethyl-3-(3'-dimethylaminopropyl)carbodiimid)/HOBT (N-Hydroxybenzotriazol) aktiviert, die Cyclisterung mit TBTU (2-(1H-Benzotriazol-1yl)-1,1,3,3-tetramethyluroniumtetrafluoroborat). Die geschützte Zuckeraminosäure 4 wird mit Z-Zu-OMe abgekürzt.

### Herstellung der Ausgangsverbindungen

### Boc-Lys(Z)-Thr-OMe

1,14 g (3 mmol) Boc-Lys(Z)-OH, 0,51 g (3 mmol) H-Thr-OMe · HCl, 0,68 g (3.6 mmol) EDCI, 0,58 g (3.6 mmol) HOBT sowie 0,66 ml (6 mmol) NMM werden bei -10° C in 10 ml THF gelöst und 15 Stunden bei Raumtemperatur gerührt. Das THF wird im Vakuum destillativ entfernt, der Rückstand in 50 ml Essigsäureethylester gelöst und die organische Phase je dreimal mit je 10 ml 0,5 N HCl, 10 % NaHCO₃-Lösung und Wasser gewaschen. Die organische Phase wird mit MgSO₄ getrocknet und das Lösungsmittel im Vakuum destillativ entfernt. Man erhält 1,36 g (92 %) dünnschichtchromatographisch einheitliches Boc-Lys(Z)-Thr-OMe als farblosen Schaum.

### Z-Phe-D-Trp-OMe

0,60 g (2 mmol) Z-Phe-OH, 0,51 g (2 mmol) H-D-Trp-OMe · HCl, 0,46 g (2.4 mmol) EDCI, 0,32 g (2.4 mmol) HOBT sowie 0,44 ml (4 mmol) NMM werden bei -10° C in 10 ml THF gelöst und 15 Stunden bei Raumtemperatur gerührt. Das THF wird im Vakuum destillativ entfernt, der Rückstand in 50 ml Essigsäureethylester gelöst und die organische Phase je dreimal mit je 10 ml 0,5 N HCl, 10 % NaHCO₃-Lösung und Wasser gewaschen. Die organische Phase wird mit MgSO₄ getrocknet und das Lösungsmittel im Vakuum destillativ entfernt. Man erhält 0,48 g (48 %) dünnschicht-chromatographisch einheitliches Z-Phe-D-Trp-OMe als farbloses Pulver.

### Boc-Lys(Z)-Thr-Zu-OMe

0,5 g (1 mmol) Boc-Lys(Z)-Thr-OMe werden in einer Mischung aus 5 ml THF und 5 ml MeOH gelöst. Unter Rühren werden 1,2 ml (1.2 mmol) einer 1N NaOH zugegeben und 2 Minuten gerührt. Die Reaktionsmischung wird mit 0,22 g (1.5 mmol) HOBT neutralisiert.
0,35 g (1 mmol) Z-Zu-OMe werden in 5 ml MeOH gelöst, 50 mg 10 % Pd/C-Katalysator zugegeben und bei Raumtemperatur unter Rühren unter einer Wasserstoff-Atmosphäre 0,5 Stunden hydriert. Der Katalysator wird abfiltriert und mit 5 ml MeOH gewaschen. Das Filtrat gibt man zu der neutralisterten Reaktionsmischung aus der Verseifung und entfernt das Lösungsmittel im Vakuum.
Der Rückstand wird in einer Mischung aus 3 ml DMF und 3 ml THF gelöst und bei -10° C 0,3 g (1.5 mmol) EDCI sowie 0,165 ml (1.5 mmol) NMM zugesetzt. Nach 15 Stunden wird die Reaktion durch Zugabe von 5,0 g Mischbettionenaustauscher (Merck Ionenaustauscher V) beendet, und 0,5 Stunden gerührt. Der Ionenaustauscher wird abfiltriert und mit MeOH gewaschen, das Filtrat im Vakuum eingeengt und an Kieselgel (Laufmittel CHCl₃ : MeOH, 4 : 1, V : V) chromatographiert.

Man erhält 0,43 g (63 %) dünnschicht-chromatographisch einheitliches Boc-Lys(Z)-Thr-Zu-OMe als farbloses Öl.

### Boc-Lys(Z)-Thr-Zu-Phe-D-Trp-OMe

0,43 g (0.63 mmol) Boc-Lys(Z)-Thr-Zu-OMe werden in einer Mischung aus 5 ml THF und 5 ml MeOH gelöst. Unter Rühren werden 0,8 ml (0.8 mmol) einer 1N NaOH zugegeben und 2 Minuten gerührt. Die Reaktionsmischung wird mit 0,15 g (1.0 mmol) HOBT neutralisiert.
0,40 g (0.8 mmol) Z-Phe-D-Trp-OMe werden in 5 ml MeOH gelöst, 50 mg 10 % Pd/C-Katalysator zugegeben und bei Raumtemperatur unter Rühren unter einer Wasserstoff-Atmosphäre 0,5 Stunden hydriert. Der Katalysator wird abfiltriert und mit 5 ml MeOH gewaschen. Das Filtrat gibt man zu der neutralisierten Reaktionsmischung aus der Verseifung und entfernt das Lösungsmittel im Vakuum.
Der Rückstand wird in einer Mischung aus 3 ml DMF und 3 ml THF gelöst und bei -10° C 0,2 g (1.0 mmol) EDCI sowie 0,165 ml (1.5 mmol) NMM zugesetzt. Nach 15 Stunden wird die Reaktion durch Zugabe von 5,0 g Mischbettionenaustauscher (Merck Ionenaustauscher V) beendet und 0,5 Stunden gerührt. Der Ionenaustauscher wird abfiltriert und mit MeOH gewaschen, das Filtrat im Vakuum eingeengt und an Kieselgel (Laufmittel CHCl₃ : MeOH, 9 : 1, V : V) chromatographiert und aus Wasser/t-Butanol lyophilisiert. Man erhält 0,51 g (80 %) dünnschicht-chromatographisch einheitliches Boc-Lys(Z)-Thr-Zu-Phe-D-Trp-OMe als farbloses Pulver.

### cyclo(-Zu¹-Phe²-D-Trp³-Lys⁴-Thr⁵)

0,2 g (0.2 mmol) Boc-Lys(Z)-Thr-Zu-Phe-D-Trp-OMe werden in 2 ml MeOH und 1 ml THF gelöst, 0,25 ml (0.25 mmol) 1N NaOH zugegeben und 3 Minuten gerührt. Es werden 200 mg stark saurer Kationenaustauscher (Amberlyst 15, H⁺-Form) zugegeben und 5 Minuten gerührt.

Der Kationenaustauscher wird abfiltriert und mit 5 ml MeOH gewaschen. Das Filtrat wird im Vakuum eingeengt und über P₂O₅ i. HV. getrocknet. Man nimmt die Substanz in 3 ml abs. THF auf, versetzt mit 0,1 ml Methanthiol und 1 ml HCl/Diethylether (abs. Diethylether wird hierzu bei 0° C mit Chlorwasserstoff gesättigt) und behandelt die Reaktionsmischung 1 Minute mit Ultraschall. Nach 5 Minuten wird das Lösungsmittel im Vakuum abgezogen und i. HV. getrocknet. Man erhält einen leicht gelblichen Feststoff der in 100 ml dest. DMF gelöst wird. Hierzu gibt man 40 µl Wasser, 40 mg (0.25 mmol) HOBT, 100 mg (0.25 mmol) TBTU sowie 170 µl DIPEA. Nach 0,5 Stunden Rühren bei Raumtemperatur wird das Lösungsmittel im Vakuum entfernt, und der Rückstand an Kieselgel (Laufmittel CHCl₃ : MeOH, 9 : 1, V : V) chromatographiert und hierauf an der HPLC gereinigt. Der so erhaltene weiße Feststoff wird in 2,5 ml MeOH gelöst, 10 mg 10 % Pd/C-Katalysator zugegeben und bei Raumtemperatur unter Rühren unter einer Wasserstoffatmosphäre 20 Minuten hydriert. Der Katalysator wird abfiltriert und mit 2 ml MeOH gewaschen. Das Lösungsmittel wird im Vakuum abdestilliert und lyophilisiert. Man erhält 82 mg (54 %) HPLC-reines cyclo(-Zu-Phe-D-Trp-Lys-Thr) als farbloses Pulver, FAB-MS: m/z 753 (M + H⁺).

Die Herstellung von cyclo(-Zu¹-Tyr²-D-Trp³-Lys⁴-Val⁵) D-22475 erfolgt auf analoge Weise. FAB-MS: m/z 767 (M + H⁺).

### Darstellung von Ac-D-(2-Nal)-p-Cl-D-Phe-D-(3-Pal)-Ser-Tyr-D-Lys(Zu)-Leu-Arg-Pro-D-AlaNH₂ · TFA D-22620

78 mg (0.22 mmol) 7-Benzoxycarbonylamino-L-glycero-L-gulo-2,6-anhydro-7-desoxy-heptonsäuremethylester werden in 2 ml MeOH gelöst und durch Zugabe von 0,23 ml 1N NaOH verseift.

Nach 5 Minuten werden 100 mg Kationenaustauscher (Aldrich, Amberlyst 15, stark sauer, H⁺-Form) zugegeben und weitere 5 Minuten gerührt. Der Ionenaustauscher wird abfiltriert und mit 5 ml MeOH gewaschen. Das Filtrat wird im Vakuum zu einem Sirup eingeengt. Dieser wird in einer Mischung aus 0,5 ml DMF und 0,5 ml THF aufgenommen und hierzu 280 mg (0.2 mmol) D-22516, 52 mg (0.25 mmol) DCC sowie 0,022 ml (0.2 mmol) N-Methylmorpholin gegeben.
Nach 12 Stunden Rühren bei Raumtemperatur gibt man die Reaktionsmischung unter Ultraschallbehandlung in 20 ml Ether. Die farblose Fällung wird abfiltriert, mit Ether gewaschen und in einer Mischung aus 10 ml MeOH und 40 ml Wasser gelöst. Das Harnstoffderivat bleibt ungelöst und wird abfiltriert. Das Filtrat wird im Vakuum zur Trockne eingeengt und in 17 ml MeOH gelöst. Diese Lösung wird in 2 ml-Portionen durch RP-Chromatographie getrennt (MPLC 30 bar, 50 g RP-Eurosilgel, Flow 10 ml/min, MeOH/H₂O mit 0,1 % TFA, Gradient 55 % MeOH auf 70 % MeOH in 40 Min.).
Das Produkt wird nach der Trennung aus Ether-Hexan gefällt. Man erhält 100 mg (0.054 mmol, 27 %) Produkt als Trifluoracetat in Form eines farblosen Pulvers.
FAB-MS: 1724.5 (M + H⁺), oben angegebene Molmasse - TFA.

Die Herstellung der Ausgangsverbindung D-22516 = Ac-D-Nal-(2)-D-Phe(4Cl)-D-Pal(3)-Ser-Tyr-D-Lys-Leu-Arg-Pro-D-AlaNH₂ ist in der Patentanmeldung von Schally et al. PCT WO 92/13883 auf Seite 33 ff angegeben.

### Darstellung des vom Nafarelin abgeleiteten LHRH-Agonisten pGlu-His-Trp-Ser-Zu-Leu-Arg-Pro-Gly-NH₂ . 2 TFA D-22677

Die Synthese wird in flüssiger Phase durchgeführt. Die Peptid-Kupplungen werden mit EDCl/HOBT aktiviert. Die geschützte Zuckeraminosäure 7-Benzoxycarbonylamino-L-glycero-L-gulo-2,6-anhydro-7-desoxy-heptonsäuremethylester wird mit Z-Zu-OMe abgekürzt.

### Synthese-Strategie:

pGlu-His(Trt) / Trp-Ser / Zu-Leu / Arg(Pmc) / Pro-Gly-NH2
Trp-Ser-Zu-Leu / Arg(Pmc)-Pro-Gly-NH2
Trp-Ser-Zu-Leu-Arg(Pmc)-Pro-Gly-NH2
pGlu-His(Trt)-Trp-Ser-Zu-Leu-Arg(Pmc)-Pro-Gly-NH2
pGlu-His-Trp-Ser-Zu-Leu-Arg-Pro-Gly-NH2

### pGlu-His(Trt)-OMe

0,39 g (3 mmol) pGlu-OH, 1,34 g (3 mmol) H-His(Trt)-OMe · HCl, 0,68 g (3.6 mmol) EDCl, 0,58 g (3.6 mmol) HOBT sowie 0,66 ml (6 mmol) NMM werden bei -10° C in 8 ml DMF gelöst und 15 Stunden bei Raumtemperatur gerührt. Das DMF wird im Vakuum destillativ entfernt, der Rückstand in 50 ml Essigsäureethylester gelöst und die organische Phase je dreimal mit je 10 ml 0,5 N HCl, 10 % NaHCO₃-Lösung und H₂O gewaschen. Die organische Phase wird mit MgSO₄ getrocknet und das Lösungsmittel im Vakuumn destillativ entfernt und das so erhaltene Rohprodukt an Kieselgel (Laufmittel CHCl₃ : MeOH 9 : 1 V : V) chromatographiert. Man erhält 0,72 g (46 %) dünnschtchtchromatographisch einheitliches pGlu-His(Trt)-OMe als farblosen harten Schaum.

### Z-Trp-Ser-OMe

3,38 g (10 mmol) Z-Trp-OH, 1,55 g (10 mmol) H-Ser-OMe · HCl, 2,00 g (10 mmol) EDCl, 1,50 g (10 mmol) HOBT sowie 3,3 ml (10 mmol) NMM werden bei -10° C in 30 ml THF gelöst und 15 Stunden bei Raumtemperatur gerührt. Das THF wird im Vakuum destillativ entfernt, der Rückstand in 50 ml Essigsäureethylester gelöst und die organische Phase je dreimal mit je 10 ml 0,5 N HCl, 10 % NaHCO₃-Lösung und H₂O gewaschen. Die organische Phase wird mit MgSO₄ getrocknet und das Lösungsmittel im Vakuum destillativ entfernt.

Der Rückstand wird in 2-Propanol aufgenommen, wobei das Produkt ausfällt. Dieses wird abfiltriert und getrocknet. Man erhält 2,90 g (68 %) dünnschichtchromatographisch einheitliches Z-Trp-Ser-OMe als farbloses Pulver.

### Z-Zu-Leu-OMe

530 mg (1.5 mmol) Z-Zu-OMe werden in 4 ml MeOH gelöst, dazu gibt man 1,6 ml 1N NaOH. Nach 5 min. ist die Verseifung beendet, man gibt 240 mg (1.6 mmol) HOBT zu, engt im Vakuum ein und kovaporiert mit Toluol. Der weiße Rückstand wird in 0,5 ml DMF gelöst, hierzu gibt man 326 mg (1.8 mmol) H-Leu-OMe · HCl, 4 ml THF, 0,20 ml (1.8 mmol) NMM sowie 310 mg (1.6 mmol) EDCl. Durch Zugabe weniger Tropfen NMM wird der pH auf 7 eingestellt und rührt 16 Stunden bei Raumtemperatur. Das Lösungsmittel wird im Vakuum entfernt und das Rohprodukt an Kieselgel (Laufmittel CHCl₃ : MeOH, 6 : 1, V : V) chromatographiert. Man erhalt 700 mg (99 %) dünnschichtchromatographisch einheitliches Z-Zu-Leu-OMe als farbloses Öl.

### Boc-Pro-Gly-NH₂

0,56 g (5 mmol) H-Gly-NH₂ · HCl werden in 20 ml MeOH gelöst und 270 mg (5 mmol) NaOMe zugegeben. Man rührt 10 min. und entfernt das Lösungsmittel im Vakuum. Zum Rückstand werden 15 ml DMF, 1,07 g (5 mmol) Boc-Pro-OH, 1,2 g (6.0 mmol) EDCl, 0,9 g (6 mmol) HOBT sowie 0,825 ml (7.5 mmol) NMM gegeben und 16 Stunden gerührt. Zur Reaktionsmischung gibt man 10 g Mischbettionenaustauscher (Merck Ionenaustauscher V), rührt 0,5 Stunden, filtriert und wäscht mit 10 ml MeOH. Das Filtrat wird im Vakuum eingeengt, das resultierende Öl in 150 ml Essigsäureethylester aufgenommen und die organische Phase je dreimal mit je 10 ml 0,5 N HCl, 10 % NaHCO₃-Lösung und H₂O gewaschen.

Die organische Phase wird mit MgSO₄ getrocknet, das Lösungsmittel im Vakuum destillativ entfernt und das Produkt aus Ether gefällt. Man erhält 0,66 g (49 %) dünnschicht-chromatographisch einheitliches Produkt Boc-Pro-Gly-NH₂ als farbloses Pulver.

### Z-Trp-Ser-Zu-Leu-OMe

0,79 g (1.8 mmol) Z-Trp-Ser-OMe werden in einer Mischung aus 2 ml THF und 2 ml MeOH gelöst. Unter Rühren werden 2,0 ml (2.0 mmol) 1N NaOH zugegeben und 2 min. gerührt.
Nach Beendigung der Verseifung (DC-Kontrolle) wird die Reaktionsmischung durch Zugabe von 0,3 g (2.0 mmol) HOBT neutralisiert.
0,70 g (1.5 mmol) Z-Zu-Leu-OMe werden in 10 ml MeOH gelöst, 100 mg 10 % Pd/C-Katalysator zugegeben und bei Raumtemperatur unter Rühren unter einer Wasserstoffatmosphäre 0,5 Stunden hydriert. Der Katalysator wird abfiltriert und mit 5 ml MeOH gewaschen. Das Filtrat gibt man zu der neutralisierten Reaktionsmischung aus der Verseifung und entfernt das Lösungsmittel im Vakuum.
Der Rückstand wird in einer Mischung aus 3 ml DMF und 3 ml THF gelöst und bei -10° C 0,34 g (1.8 mmol) EDCl sowie 0,165 ml (1.5 mmol) NMM zugesetzt. Nach 15 Stunden wird die Reaktion durch Zugabe von 10,0 g Mischbettionenaustauscher (Merck Ionenaustauscher V) beendet und 0,5 Stunden gerührt. Der Ionenaustauscher wird abfiltriert und mit MeOH gewaschen, das Filtrat im Vakuum eingeengt und an Kieselgel (Laufmittel CHCl₃ : MeOH, 9 : 1, V : V) chromatographiert. Man erhält 0,74 g (63 %) dünnschicht-chromatographisch einheitliches Z-Trp-Ser-Zu-Leu-OMe als farblosen Feststoff.

### Fmoc-Arg(Pmc)-Pro-Gly-NH₂

540 mg (2.0 mmol) Boc-Pro-Gly-NH₂ werden in 5 ml abs. THF gelöst und unter Rühren mit 3 ml HCl/Diethylether (abs. Diethylether wird hierzu bei 0° C mit Chlorwasserstoff gesättigt) versetzt. Nach 0,5 Stunden wird das Lösungsmittel im Vakuum entfernt und dreimal mit je 5 ml Toluol kovaporiert. Man gibt 110 mg (2.0 mmol) NaOMe in 5 ml abs. THF zu und nach 5 min. 5 ml DMF, 300 mg (2.0 mmol) HOBT sowie 1,32 g Fmoc-Arg(Pmc)-OH. Man kühlt auf -10° C, gibt 600 mg (3 mmol) EDCI zu und stellt mit 0,33 ml NMM einen pH von 7,0 ein. Nach 15 Stunden Rühren wird das Lösungsmittel im Vakuum entfernt, der resultierende Sirup in 150 ml Essigsäureethylester aufgenommen und die organische Phase je dreimal mit je 10 ml 0,5 N HCl, 10 % NaHCO₃-Lösung und H₂O gewaschen. Die organische Phase wird mit MgSO₄ getrocknet, das Lösungsmittel im Vakuum destillativ entfernt und das Produkt aus Hexan gefällt.
Man erhält 1,52 g (93 %) dünnschicht-chromatographisch einheitliches Fmoc-Arg(Pmc)-Pro-Gly-NH₂ als farbloses Pulver.

### Z-Trp-Ser-Zu-Leu-Arg(Pmc)-Pro-Gly-NH₂

0,59 g (0.8 mmol) Z-Trp-Ser-Zu-Leu-OMe werden in einer Mischung aus 1 ml THF und 2 ml MeOH gelöst. Unter Rühren werden 1,0 ml (1.0 mmol) 1N NaOH zugegeben und 5 min. gerührt. Nach Beendigung der Verseifung (DC-Kontrolle) wird die Reaktionsmischung durch Zugabe von 0,15 g (1.0 mmol) HOBT neutralisiert und das Lösungsmittel im Vakuum entfernt.
0,62 g (0.76 mmol) Fmoc-Arg(Pmc)-Pro-Gly-NH₂ werden in 5 ml CH₂Cl₂ gelöst, 5 ml Dimethyl-ethylamin zugegeben und über Nacht gerührt. Aus der Reaktionsmischung wird das Lösungsmittel destillativ im Vakuum entfernt und der pulvrige Rückstand in 3 ml DMF aufgenommen.

Die Reaktionsmischung wird zur Trockne eingeengt und der Rückstand in 3 ml DMF gelöst. Die Lösung gibt man zu dem neutralisierten Ansatz aus der Verseifung und 3 ml THF hinzu. Bei -10° C 0,16 g (0.8 mmol) EDCI sowie 0,154 ml (1.4 mmol) NMM zugesetzt. Nach 8 Stunden wird die Reaktionsmischung im Vakuum eingeengt und an Kieselgel (Laufmittel CHCl₃ : MeOH, 4 : 1, V : V) chromatographiert. Man erhält 0,50 g (50 %) dünnschichtchromatographisch einheitliches Z-Trp-Ser-Zu-Leu-Arg-(Pmc)-Pro-Gly-NH₂ als farblosen Feststoff.

### pGlu-His(Trt)-Trp-Ser-Zu-Leu-Arg(Pmc)-Pro-Gly-NH₂

162 mg (0.31 mmol) pGlu-His(Trt)-OMe werden in einer Mischung aus 1 ml THF und 1 ml MeOH gelöst. Unter Rühren werden 0,5 ml (0.5 mmol) 1N NaOH zugegeben und 0,5 Stunden gerührt. Nach Beendigung der Verseifung (DC-Kontrolle) wird die Reaktionsmischung durch Zugabe von 75 mg (0.5 mmol) HOBT neutralisiert.
367 mg (0.28 mmol) Z-Trp-Ser-Zu-Leu-Arg(Pmc)-Pro-Gly-NH₂ werden in 5 ml MeOH gelöst, 50 mg 10 % Pd(C-Katalysator zugegeben und bei Raumtemperatur unter Rühren unter einer Wasserstoffatmosphäre 0,5 Stunden hydriert. Der Katalysator wird abfiltriert und mit 5 ml MeOH gewaschen. Das Filtrat gibt man zu der neutralisierten Reaktionsmischung aus der Verseifung und entfernt das Lösungsmittel im Vakuum. Der Rückstand wird in einer Mischung aus 1 ml DMF und 3 ml THF gelöst und bei -10° C 60 mg (0.31 mmol) EDCI sowie 33 µl (0.31 mmol) NMM zugesetzt. Nach 15 Stunden Rühren wird die Reaktionsmischung im Vakuum eingeengt und an Kieselgel (Laufmittel CHCl₃ : MeOH, 4 : 1, V : V) chromatographiert.
Man erhält 0,31 g (63 %) dünnschicht-chromatographisch einheitliches pGlu-His(Trt)-Trp-Ser-Zu-Leu-Arg(Pmc)-Pro-Gly-NH₂ als farblosen Feststoff.

### pGlu-His-Trp-Ser-Zu-Leu-Arg-Pro-Gly-NH₂ · 2 TFA D-22677

### MG 1379.45 g/mol

0,20 g (0.13 mmol) pGlu-His(Trt)-Trp-Ser-Zu-Leu-Arg-(Pmc)-Pro-Gly-NH₂ werden in einer zuvor hergestellten Mischung aus 8.25 g TFA, 0,5 g H₂O, 0,5 g Phenol, 0,5 g Thioanisol und 0,25 g Methanthiol gelöst und 4 Stunden bei Raumtemperatur gerührt. Die Reaktionsmischung wird in eine Mischung aus 200 ml Diethylether und 50 ml Hexan gegeben, wobei das Peptid ausfällt. Dieses wird abfiltriert und mit Diethylether gewaschen. Das Peptid wird durch RP-Chromatographie gereinigt (HPLC, Nucleosil-Gel, Flow 6 ml/min., CH₃CN/H₂O mit 0,1 % TFA, Gradient 20 % CH₃CN auf 60 % CH₃CN in 30 min.). Das Produkt wird nach der Trennung aus Ether-Hexan gefällt.
Man erhält 120 mg (0.087 mmol, 67 %) Produkt als Di-Trifluoracetat in Form eines farblosen Pulvers.
FAB-MS: 1151.6 (M + H⁺), oben angegebene Molmasse - 2 · TFA.

### Darstellung des vom Cetrorelix abgeleiteten LHRH-Antagonisten Ac-D-(2-Nal)-p-Cl-D-Phe-D-(3-Pal)-Ser-Tyr-Zu-Leu-Arg(TFA)-Pro-D-Ala-NH₂ D-23010

Die Synthese wird in flüssiger Phase durchgeführt. Die Peptid-Kupplungen werden mit EDCl/HOBT bzw. HOOBT aktiviert. Die geschützte Zuckeraminosäure wird wie im vorhergehenden Beispiel mit Z-Zu-OMe abgekürzt.

### Synthesestrategie:

Ac-D-(2-Nal)-p-Cl-D-Phe-D-(3-Pal) / Ser-Tyr / Zu-Leu / Arg(HCl)-Pro-D-Ala-NH2
Ser-Tyr-Zu-Leu
Ac-D-(2-Nal)-p-Cl-D-Phe-D-(3-Pal)-Ser-Tyr-Zu-Leu
Ac-D-(2-Nal)-p-Cl-D-Phe-D-(3-Pal)-Ser-Tyr-Zu-Leu-Arg(TFA-Pro-D-Ala-NH2

### Z-Ser-Tyr-OET

0,72 g (3 mmol) Z-Ser-OH, 0,74 g (3 mmol ) H-Tyr-OET · HCl, 0,68 g (3.6 mmol) EDCl, 0,58 g (3.6 mmol) HOBT sowie 0,66 ml (6 mmol) NMM werden bei -10° C in 8 ml DMF gelöst und 15 Stunden bei Raumtemperatur gerührt. Das DMF wird im Vakuum destillativ entfernt, der Rückstand in 50 ml Essigsäureethylester gelöst und die organische Phase je dreimal mit je 10 ml 0,5 N HCl, 10 % NaHCO₃-Lösung und H₂O gewaschen. Die organische Phase wird mit MgSO₄ getrocknet und das Lösungsmittel im Vakuum destillativ entfernt. Man erhält 1,21 g (94 %) dünnschicht-chromatographisch einheitliches Z-Ser-Tyr-OET als farblosen Feststoff.

Die Synthese von Z-Zu-Leu-OMe ist im vorstehenden Beispiel beschrieben.

### Z-Ser-Tyr-Zu-Leu-OMe

0,32 g (0.75 mmol) Z-Ser-Try-OEt werden in einer Mischung aus 2 ml THF und 2 ml MeOH gelöst. Unter Rühren werden 1,0 ml (1.0 mmol) 1N NaOH zugegeben und 5 min. gerührt. Nach Beendigung der Verseifung (DC-Kontrolle) wird die Reaktionsmischung durch Zugabe von 0,15 g (1.0 mmol) HOBT neutralisiert.
0,24 g (0.5 mmol) Z-Zu-Leu-OMe werden in 5 ml MeOH gelöst, 50 mg 10 % Pd/C-Katalysator zugegeben und bei Raumtemperatur unter Rühren unter einer Wasserstoffatmosphäre 0,5 Stunden hydriert. Der Katalysator wird abfiltriert und mit 5 ml MeOH gewaschen. Das Filtrat gibt man zu der neutralisierten Reaktionsmischung aus der Verseifung und entfernt das Lösungsmittel im Vakuum.
Der Rückstand wird in einer Mischung aus 2 ml DMF und 2 ml THF gelöst und bei -10° C 0,15 g (0.75 mmol) EDCl sowie 0,11 ml (1.0 mmol) NMM zugesetzt. Nach 15 Stunden wird die Reaktionslösung im Vakuum eingeengt und an Kieselgel (Laufmittel CHCl₃ : MeOH, 6 : 1, V : V) chromatographiert. Man erhält 0,26 g (72 %) dünnschichtchromatographisch einheitliches Z-Ser-Tyr-Zu-Leu-OMe als farblosen Feststoff.

### Ac-D-(2-Nal)-p-Cl-D-Phe-D-(3-Pal)-Ser-Tyr-Zu-Leu-OMe

145 mg (0.2 mmol) Z-Ser-Tyr-Zu-Leu-OMe werden in 5 ml MeOH gelöst und mit einer Spatelspitze Pd/C-Katalysator (10 %ig) 0,5 Stunden unter einer Wasserstoffatmosphäre gerührt. Der Katalysator wird abfiltriert, mit MeOH gewaschen und das Filtrat im Vakuum zur Trockne eingeengt. Der Rückstand wird in 3 ml DMF gelöst, 180 mg (0.3 mmol) Ac-D-Nal-D-p-Cl-Phe-D-Pal-OH zugegeben, 60 mg (0.3 mmol) EDCl, 45 mg (0.3 mmol) HOOBT sowie 33 µl (0.3 mmol) NMM zugegeben und über Nacht gerührt. Die Reaktionsmischung wird i. HV. eingeengt und der Rückstand an Kieselgel (Laufmittel CHCl₃/MeOH 4 : 1) chromatographiert. Man erhält 187 mg (80 %) chromatographisch einheitliches Ac-D-(2-Nal)-p-Cl-D-Phe-D-(3-Pal)-Ser-Tyr-Zu-Leu-OMe als farblosen harten Schaum.

### Ac-D-(2-Nal)-p-Cl-D-Phe-D-(3-Pal)-Ser-Tyr-Zu-Leu-Arg-(TFA)-Pro-D-Ala-NH₂ D-23010

187 mg (0.2 mmol) Ac-D-(2-Nal)-p-Cl-D-Phe-D-(3-Pal)-Ser-Tyr-Zu-Leu-OMe werden in 2 ml MeOH und 2 ml THF und 0,3 ml 1N NaOH zugegeben und 0,5 Stunden bei Raumtemperatur gerührt. Ist die Verseifung beendet (DC-Kontrolle), wird durch Zugabe von HOOBT ein pH von 4 - 5 eingestellt und im Vakuum eingeengt. Der Rückstand wird in 2 ml DHF gelöst, 110 mg (0.3 mmol) H-Arg(HCl)-Pro-D-Ala-NH₂ und 60 mg (0.3 mmol) EDCl zugegeben und über Nacht gerührt. (Der pH der Reaktionslösung sollte bei 5 liegen).

Die Reaktionsmischung wird im Vakuum eingeengt und der Rückstand an Kieselgel (Laufmittel CH₃CN/H₂O 4 : 1) chromatographiert. Man erhält 240 mg (80 %) chromatographisch weitgehend einheitliches Produkt, welches durch Zugabe von wenig HCl ins Hydrochlorid überführt wird. Die weitere Reinigung erfolgt durch RP-Chromatographie (HPLC, Nucleosil-Gel), Flow 6 ml/min, CH₃CN/H₂O mit 0,1 % TFA, Gradient 35 % CH₃CN auf 40 % CH₃CN in 30 min). Das Produkt wird aus t-Butanol lyophilisiert. Man erhält 90 mg (0.060 mmol, 30 %) Produkt als Trifluoracetat in Form eines farblosen Pulvers.

## Patentansprüche

1. Verwendung von D-Glucopyranuronsäuren der Formel, 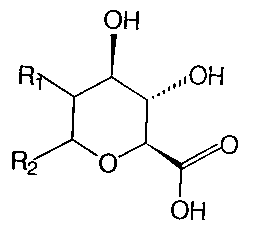 wobei
R₁ = OH, OCH₃, NH₂, NH-CO-O-CH₂-C₆H₅, NH-CO-O-tert.butyl, NH-CO-CH₃ und entsprechende Schutzgruppen
R₂ = CH₂-NH₂, OH, OCH₃, OCH₂-C₆H₅, CH₂-NH-CO-O-CH₂-C₆O₅, NH-CO-O-tert.butyl und entsprechende Schutzgruppen
bedeuten, der enantiomeren Formen und deren Derivate zum Einbau in pharmakologisch wirksame Peptide und deren Salze.

2. 7-Amino-L-glycero-L-gulo-2,6-anhydro-7-desoxy-heptonsäure der Formel

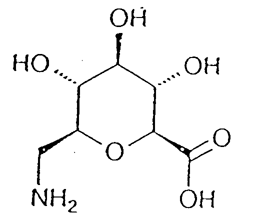

3. Verfahren zur Herstellung von 7-Amino-L-glycero-L-gulo-2,6-anhydro-7-desoxy-heptonsäure durch
a) Reduktion von β-D-Glucopyranosylnitromethan mit Wasserstoff an einem Palladium Katalysator und anschließender Umsetzung mit Chloramelsensäurebenzylester
b) Oxidation von N-Benzoxycarbonyl-β-D-glucopyranosylmethylamin mit Sauerstoff an einem Platin-Katalysator und anschließender Veresterung mit Alkohol
c) und Abspaltung der Schutzgruppen des 7-Benzoxycarbonylamino-L-glycero-L-gulo-2,6-anhydro-7-desoxy-heptonsäuremethylesters in an sich bekannter Weise

4. 7-Benzoxycarbonylamino-L-glycero-L-gulo-2,6-anhydro-7-desoxy-heptonsäuremethylester

5. 7-Acetylamino-L-glycero-L-gulo-2,6-anhydro-7-desoxy-heptonsäuremethylester

6. 2-Benzoxycarbonylamino-O¹-methyl-2-desoxy-β-D-glucopyranuronsäure und deren geschützte Derivate

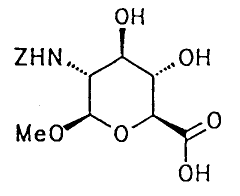

7. Verwendung von 7-Amino-L-glycero-L-gulo-2,6-anhydro-7-desoxy-heptonsäure und der enantiomeren 7-Amino-D-glycero-D-gulo-2,6-anhydro-7-desoxy-heptonsäure zum Einbau in pharmakologisch wirksame Peptide und deren Salze.

8. Verwendung von 2-Benzoxycarbonyl amino-O¹-methyl-2-desoxy-β-D-glucopyranuronsäure und der enantiomeren 2-Benzoxycarbonylamino-O¹-methyl-2-desoxy-α-D-glucopyranuronsäure zum Einbau in pharmakologisch wirksame Peptide und deren Salze.

9. Pharmakologisch wirksame Peptide und deren Salze enthaltend als Baustein 7-Amino-L-glycero-L-gulo-2,6-anhydro-7-desoxy-heptonsäure oder ein entsprechend modifiziertes Derivat.

10. Pharmakologisch wirksame Peptide und deren Salze enthaltend als Baustein 2-Amino-O¹-methyl-2-desoxy-D-glucopyranuronsäure oder ein entsprechend modifiziertes Derivat.

11. Ac-D-(2-Nal)-p-Cl-D-Phe-D-(3-Pal)-Ser-Tyr-D-lys-(Zu)-Leu-Arg-Pro-D-Ala-NH₂ x TFA
Zu = 7-Amino-L-glycero-L-gulo-2,6-anhydro-7-desoxyheptonsäure
TFA = Trifluoracetat

12. pGlu-His-Trp-Ser-Zu-Leu-Arg-Pro-Gly-NH₂ x 2TFA, wobei Zu und TFA wie im vorhergehenden Anspruch bedeuten.

13. Ac-D-(2-Nal)-p-Cl-D-Phe-D-(3-Pal)-Ser-Tyr-Zu-Leu-Arg(TFA)-Pro-D-Ala-NH₂, wobei Zu und TFA wie im vorhergehenden Anspruch bedeuten.

14. Ac-D-(2-Nal)-p-Cl-D-Phe-D-(3-Pal)-Ser-Zu-Leu-Arg-(TFA)-Pro-D-Ala-NH₂, wobei Zu und TFA wie im vorhergehenden Anspruch bedeuten.

15. Verwendung einer Verbindung nach den Ansprüchen 11, 12, 13 oder 14 zur Herstellung eines Arzneimittels.
